# EUROPÄISCHE PATENTSCHRIFT

(11) **EP 1 028 105 B1**
(45) Veröffentlichungstag und Bekanntmachung des Hinweises auf die Patenterteilung: **21.04.2004**
(21) Anmeldenummer: 00102087.4
(22) Anmeldetag: 03.02.2000
(51) Int. Cl.: C07C 253/30, C07C 255/19

(54) **Verfahren zur Herstellung von Cyanessigsäureestern**
Process for the preparation of Cyanoacetic esters
Procédé pour la préparation d'ester de l'acide cyanoacétique

(30) Priorität: 09.02.1999 EP 99102286
(43) Veröffentlichungstag der Anmeldung: 16.08.2000
(73) Patentinhaber: Lonza AG, 4052 Basel (CH)
(72) Erfinder: Hanselmann, Paul Dr., 3902 Brig-Glis (CH); Hildbrand, Stefan Dr., 3930 Visp (CH)

(56) Entgegenhaltungen:
- US-A- 4 174 347
- LHOMMET, GERARD ET AL: "Preparation of benzyl esters by phase transfer catalysis" C. R. SEANCES ACAD. SCI., SER. C (1980), 290(23), 445-7 , XP000907001

## Beschreibung

Die Erfindung betrifft ein Verfahren zur Herstellung von Cyanessigsäureestem der allgemeinen Formel 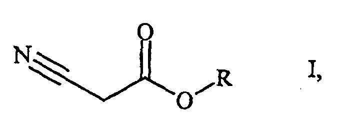 worin R C₁₋₁₀-Alkyl oder C₃₋₁₀-Alkenyl bedeutet.

Unter C₁₋₁₀-Alkyl sind hier und im folgenden alle linearen oder verzweigten primären, sekundären oder tertiären Alkylgruppen mit 1 bis 10 Kohlenstoffatomen zu verstehen, insbesondere Gruppen wie Methyl, Ethyl, Propyl, Isopropyl, Butyl, Isobutyl, *sec*-Butyl, *tert*-Butyl, Pentyl, Hexyl, Heptyl, Octyl, Nonyl oder Decyl.
Unter C₃₋₁₀-Alkenyl sind entsprechende Gruppen mit 3 bis 10 Kohlenstoffatomen und mindestens einer C=C-Doppelbindung zu verstehen, wobei die Doppelbindung vorteilhaft durch mindestens ein gesättigtes Kohlenstoffatom von der freien Valenz getrennt ist. Hierunter fallen insbesondere Gruppen wie Allyl, Methallyl, But-2-enyl (Crotyl), But-3-enyl und so weiter.

Die übliche Synthese von Cyanessigsäureestern erfolgt durch Cyanidierung von Chloressigsäure-Natriumsalz in wässriger Lösung, gefolgt von einer säurekatalysierten Veresterung mit dem entsprechenden Alkohol, wobei das gebildete Wasser azeotrop abdestilliert wird. Ein wesentlicher Nachteil dieses zweistufigen Verfahrens ist, dass nach der Cyanidierung das Wasser entfernt werden muss, weil die anschliessende Veresterung nur unter weitgehend wasserfreien Bedingungen möglich ist. Grosstechnisch geschieht dies normalerweise durch Eindampfen des Wassers.

Da das als Zwischenprodukt gebildete Cyanessigsäure-Natriumsalz zudem sehr gut wasserlöslich ist, ist eine Methode zu dessen Veresterung in Wasser als Lösungsmittel wünschenswert.

US-A-4 174 347 offenbart ein Verfahren zur Herstellung von Cyanessigsäureestern aus Alkalicyanacetaten und aliphatischen oder cycloaliphatischen Halogenkohlenwasserstoffen in Anwesenheit von Phasentransferkatalysatoren, aber in Abwesenheit von Wasser. G. Lhommet et al. (*C. R. Seances Acad. Sci., Ser. C* **1980,** *290*, 445-447) beschreiben u.a. die Herstellung von Benzylcyanacetat aus Kaliumcyanacetat und Benzylbromid in Gegenwart von Tetrabutylammoniumchlorid in Wasser/Chloroform.

Aufgabe der vorliegenden Erfindung war daher, ein Verfahren zu entwickeln, bei dem die nach der Cyanidierung anfallende wässrige Lösung von Cyanessigsäure-Natriumsalz direkt verestert werden kann.

Erfindungsgemäss wird diese Aufgabe durch das Verfahren nach Patentanspruch 1 gelöst.

Es wurde gefunden, dass Cyanessigsäureester der allgemeinen Formel 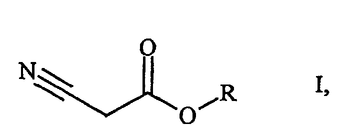 worin R C₁₋₁₀-Alkyl, oder C₃₋₁₀-Alkenyl bedeutet, dadurch hergestellt werden können, dass Natriumcyanacetat in Form der bei der Umsetzung von Natriumchloracetat mit Natriumcyanid anfallenden wässrigen Lösung in einem wässrig/organischen Zweiphasensystem in Gegenwart eines Phasentransferkatalysators mit einem Halogenid der allgemeinen Formel R-X (II), worin R die oben genannte Bedeutung hat und X Chlor, Brom oder Iod ist, umgesetzt wird. Als organische Phase kann hierbei das Halogenid (II) allein oder im Gemisch mit einem organischen Lösungsmittel fungieren.

X ist vorzugsweise Chlor oder Brom.

Als Phasentransferkatalysator wird vorzugsweise ein quartäres Ammoniumsalz eingesetzt. Besonders bevorzugte quartäre Ammoniumsalze sind die Tetra-*n*-C₄₋₁₀-alkylammonium-, Benzyltri-*n*-C₁₋₈-alkylammonium- oder Methyltri-*n*-C₄₋₁₀-alkylammoniumhalogenide, insbesondere die Chloride und Bromide.

Ebenfalls besonders bevorzugt ist die Verwendung von *tert*-Butylmethylether oder Chlorbenzol als Lösungsmittel in der organischen Phase.

Die folgenden Beispiele verdeutlichen die Durchführung des erfindungsgemässen Verfahrens, ohne dass darin eine Einschränkung zu sehen ist. Sämtliche Reaktionen wurden in einem Autoklaven mit ca. 250 ml Innenvolumen durchgeführt. Die Bestimmung der Ausbeute erfolgte gaschromatographisch mit Hilfe eines internen Standards.

### Beispiel 1

### Cyanessigsäuremethylester

Zu einem Gemisch von 1,70 g (0,02 mol) Cyanessigsäure, 0,8 g (0,02 mol) Natriumhydroxid und 0,64 g (2,0 mmol) Tetrabutylammoniumbromid in 15 ml *tert*-Butyl-methylether/Wasser 2:1 wurden 10,0 g (9,9 Äquiv., 0,20 mol) Methylchlorid eingeleitet. Das Reaktionsgemisch wurde innerhalb von 30 min auf 100 °C Innentemperatur geheizt (110°C Ölbadtemperatur), wobei der Druck im Autoklaven von 4 auf 10 bar anstieg. Nach 3,5 h bei 100 °C wurde abgekühlt und entspannt. Die wässrige Phase wurde mit 3,10 g 1 M Natronlauge von pH 2,9 auf 5,9 gestellt, die organische Phase wurde abgetrennt und die wässrige Phase mit *tert*-Butylmethylether (2×6 ml) extrahiert. Die vereinigten organischen Phasen wurden mit Natriumsulfat getrocknet, mit Bernsteinsäuredimethylester (als interner Standard) versetzt und gaschromatographisch analysiert. Es wurden 1,36 g (68%) Cyanessigsäuremethylester erhalten.

### Vergleichsbeispiel 1

### Cyanessigsäuremethylester

Es wurde verfahren wie in Beispiel 1 beschrieben, jedoch ohne Zusatz von Tetrabutylammoniumbromid. Die Ausbeute an Cyanessigsäuremethylester betrug nur 13%.

### Beispiel 2

### Cyanessigsäureethylester

Ein Gemisch von 1,70 g (0,02 mol) Cyanessigsäure, 0,8 g (0,02 mol) Natriumhydroxid, 10,90 g (0,10 mol, 5 Äquiv.) Ethylbromid und 0,64 g (2,0 mmol) Tetrabutylammoniumbromid in 15 ml Chlorbenzol/Wasser (2:1) wurde innerhalb von 30 min auf 100 °C Innentemperatur geheizt und 3,5 h bei 100 °C (110 °C Ölbadtemperatur) gerührt. Anschliessend wurde das Reaktionsgemisch abgekühlt, die Phasen wurden getrennt und die wässrige Phase (pH = 6,85) wurde mit *tert*-Butylmethylether (2×5 ml) extrahiert. Die vereinigten organischen Phasen wurden mit Natriumsulfat getrocknet, mit Bemsteinsäuredimethylester (als interner Standard) versetzt und gaschromatographisch analysiert. Es wurden 1,46 g (65%) Cyanessigsäureethylester erhalten.

## Patentansprüche

1. Verfahren zur Herstellung von Cyanessigsäureestern der allgemeinen Formel 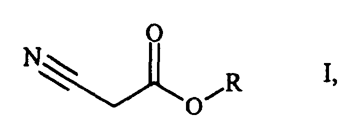 worin R C₁₋₁₀-Alkyl, oder C₃₋₁₀-Alkenyl bedeutet, **dadurch gekennzeichnet, dass** Natriumcyanacetat in Form der bei der Umsetzung von Natriumchloracetat mit Natriumcyanid anfallenden wässrigen Lösung in einem wässrig/organischen Zweiphasensystem in Gegenwart eines Phasentransferkatalysators mit einem Halogenid der allgemeinen Formel R-X (II), worin R die oben genannte Bedeutung hat und X Chlor, Brom oder Iod ist, umgesetzt wird.

2. Verfahren nach Anspruch 1, **dadurch gekennzeichnet, dass** X Chlor oder Brom ist.

3. Verfahren nach Anspruch 1 oder 2, **dadurch gekennzeichnet, dass** als Phasentransferkatalysator ein quartäres Ammoniumsalz eingesetzt wird.

4. Verfahren nach Anspruch 3, **dadurch gekennzeichnet, dass** als quartäres Ammoniumsalz ein Tetra-*n*-C₄₋₁₀-alkytammonium-, Benzyltri-*n*-C₁₋₈-alkylammonium- oder Methyltri-*n*-C₄₋₁₀-alkylammoniumhalogenid, vorzugsweise Chlorid oder Bromid, eingesetzt wird.

5. Verfahren nach einem der Ansprüche 1 bis 4, **dadurch gekennzeichnet, dass** die organische Phase *tert*-Butylmethylether oder Chlorbenzol als Lösungsmittel enthält.

## Claims

1. Process for preparing cyanoacetic esters of the general formula 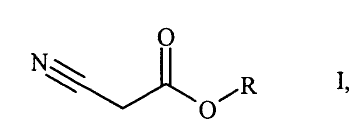 in which R is C₁₋₁₀-alkyl or C₃₋₁₀-alkenyl, **characterized in that** sodium cyanoacetate in the form of the aqueous solution obtained in the reaction of sodium chloroacetate with sodium cyanide is reacted in an aqueous/organic two-phase system in the presence of a phase-transfer catalyst with a halide of the general formula R―X (II), in which R is as defined above and X is chlorine, bromine or iodine.

2. Process according to Claim 1, **characterized in that** X is chlorine or bromine.

3. Process according to Claim 1 or 2, **characterized in that** the phase-transfer catalyst used is a quaternary ammonium salt.

4. Process according to Claim 3, **characterized in that** the quaternary ammonium salt used is a tetra-*n*-C₄₋₁₀-alkylammonium, benzyltri-*n*-C₁₋₈-alkylammonium or methyltri-*n*-C₄₋₁₀-alkylammonium halide, preferably chloride or bromide.

5. Process according to any of Claims 1 to 4, **characterized in that** the organic phase comprises *tert*-butyl methyl ether or chlorobenzene as solvent.

## Revendications

1. Procédé pour la préparation d'esters d'acide cyanoacétique de formule générale 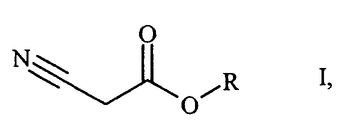 dans laquelle R est un groupe alkyle en C₁₋₁₀ ou alcényle en C₃₋₁₀, **caractérisé en ce qu'**on fait réagir du cyanoacétate de sodium sous forme de la solution aqueuse formée lors de la réaction du chloracétate de sodium avec du cyanure de sodium, dans un système biphasique aqueux/organique, en présence d'un catalyseur de transfert de phase, avec un halogénure de formule générale R-X (II), dans laquelle R a la signification donnée plus haut et X est le chlore, le brome ou l'iode.

2. Procédé selon la revendication 1, **caractérisé en ce que** X est le chlore ou le brome.

3. Procédé selon la revendication 1 ou 2, **caractérisé en ce qu'**on utilise comme catalyseur de transfert de phase un sel d'ammonium quaternaire.

4. Procédé selon la revendication 3, **caractérisé en ce qu'**on utilise en tant que sel d'ammonium quaternaire un halogénure, de préférence un chlorure ou bromure, de tétra-*n*-alkyl(C₄₋₁₀)-ammonium, benzyltri-*n*-alkyl(C₁₋₈)ammonium ou méthyltri-*n*-alkyl(C₄₋₁₀)ammonium.

5. Procédé selon l'une quelconque des revendications 1 à 4, **caractérisé en ce que** la phase organique contient en tant que solvant de l'oxyde de méthyle et de *tert*-butyle ou du chlorobenzène.
